(19) Europäisches Patentamt
European Patent Office
Office européen des brevets

(11) **EP 4 220 323 A1**

(12) **EUROPEAN PATENT APPLICATION**

(43) Date of publication:
**02.08.2023 Bulletin 2023/31**

(21) Application number: **22154269.9**

(22) Date of filing: **31.01.2022**

(51) International Patent Classification (IPC):
**G05B 23/02** (2006.01)    **G06Q 10/00** (2023.01)
**G06N 20/00** (2019.01)    **G16H 40/40** (2018.01)

(52) Cooperative Patent Classification (CPC):
**G05B 23/0283; G05B 23/0294; G06N 20/00;
G06Q 10/20; G16H 40/40;** G05B 23/0216;
G05B 2219/2652

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Koninklijke Philips N.V.
5656 AG Eindhoven (NL)**

(72) Inventors:
• **BEREZHNYY, Igor**
  **Eindhoven (NL)**
• **JOHNSON, Mark Thomas**
  **Eindhoven (NL)**
• **SISODIA, Rajendra Singh**
  **Eindhoven (NL)**
• **PAUL, Soubhik**
  **Eindhoven (NL)**
• **SREENIVASAN, Rithesh**
  **Eindhoven (NL)**

(74) Representative: **Philips Intellectual Property &
Standards
High Tech Campus 52
5656 AG Eindhoven (NL)**

(54) **PERFORMING A MAINTENANCE OPERATION ON A MEDICAL IMAGING SYSTEM**

(57) The disclosure relates to a computer-implemented method of performing a maintenance operation on a medical imaging system in response to a performance issue. The method includes extracting one or more issue features from input data, and selecting, based on the extracted one or more issue features, a maintenance task template for resolving the performance issue. The maintenance task template is selected from a database of maintenance task templates, and each maintenance task template defines one or more maintenance operations to be performed on the medical imaging system to resolve performance issues. The one or more maintenance operations defined in the selected maintenance task template are executed on the medical imaging system, in order to resolve the medical imaging system performance issue.

FIG. 2

**Description**

FIELD OF THE INVENTION

The disclosure relates to performing maintenance operations on medical imaging systems.

BACKGROUND OF THE INVENTION

Medical imaging systems such as X-ray, computed tomography, positron emission tomography "PET", single photon emission computed tomography "SPECT", and magnetic resonance "MR" imaging systems occasionally require maintenance in response to a performance issue. A performance issue, i.e. a degradation in performance, may occur in the form of a reduction in the image quality of images generated by the imaging system, an increase in the time required to complete various operations such as the reconstruction of images or to position moving parts of the imaging system, and so forth. In some cases a performance issue is observed by a user. In other cases, error messages that are indicative of the performance issue may be generated automatically by the imaging system and displayed to a user or stored in a log file. In either event, the operator of such imaging systems simply desires that the performance of the imaging system is restored to an acceptable level, and in an efficient manner, in order to minimize the risk of the performance issue disrupting clinical workflow.

Such performance issues are typically reported to a medical imaging system service facility for investigation by a service engineer, whereupon the service engineer commences an initial investigation in order to establish a root cause of the issue. Establishing the root cause of such issues is however far from trivial because of the complex nature of medical imaging systems. A user typically only observes the symptoms of the issue, and so the user might only describe the issue to the service engineer at high-level. If error messages are generated by the imaging system, these can assist the service engineer to narrow-down the root cause of the issue. However, error messages typically lack specificity, and often fail to point to a specific root cause. A result of such initial investigations is therefore typically a list of potential root causes, some of which may require further investigation, and a list of associated maintenance operations such as diagnostic tests, mechanical checks, adjustments to operating parameters of the imaging system, software updates, instructions to replace components of the imaging system, and so forth, in order to establish the actual root cause and to ultimately resolve the performance issue.

As a result, a service engineer tasked with resolving a performance issue may be faced with multiple options for different potential root causes to investigate, different options for ways in which to investigate each potential root cause, and similarly, different options for ways in which to resolve the performance issue. Determining which course of action to pursue in resolving the performance issue can therefore be challenging.

Accordingly, there is therefore a need for improvements in the way in which maintenance operations are performed on medical imaging systems in order to resolve performance issues.

SUMMARY OF THE INVENTION

The invention is defined by the claims.

According to examples in accordance with an aspect of the present disclosure, there is provided a computer-implemented method of performing a maintenance operation on a medical imaging system in response to a performance issue. The method comprises:

- receiving input data representing the performance issue;
- extracting one or more issue features from the input data, the extracted issue features including one or more of: a type of process executed on or by the medical imaging system prior to a time of the performance issue, an identification of a subcomponent installed in the medical imaging system, an amount of wear of a component of the imaging system, sensor data from the medical imaging system, a history of previous maintenance operations or software updates performed on the medical imaging system, and an expected time within which the performance issue must be resolved;
- selecting, based on the extracted one or more issue features, a maintenance task template for resolving the performance issue, wherein the maintenance task template is selected from a database of maintenance task templates, each maintenance task template defining one or more maintenance operations to be performed on the medical imaging system to resolve performance issues; and
- executing the one or more maintenance operations defined in the selected maintenance task template on the medical imaging system, in order to resolve the medical imaging system performance issue.

The use of a maintenance task template provides an efficient means of resolving the performance issue. Since the maintenance task template is selected based on the extracted issue features, an optimal appropriate maintenance task template may be selected for resolving the performance issue. Moreover, it permits the performance issues that have similar issue features to be resolved in a similar manner. As a consequence, the maintenance operation may be resolved within a predictable timeframe, which helps to minimize the impact of the performance issue on clinical workflow.

In an example, the operation of executing the one or more maintenance operations comprises performing a diagnostic test on the medical imaging system, and adjusting one or more operating parameters of the medical imaging system in response to a result of the diagnostic test. Examples of diagnostic tests that may be performed in the maintenance operation include reading out the values of various sensors, such as an image, vibration, or temperature sensor in the medical imaging system, and so forth. Examples of parameters of the medical imaging system that may be adjusted in the maintenance operation include an operating voltage of a component of the imaging system such as an X-ray tube, an offset or a gain of amplifiers in an image sensor in the medical imaging system, and image reconstruction parameters, may be adjusted in this operation. The result of the maintenance operation may be to resolve the performance issue, or alternatively to confirm that a potential root cause is not actually responsible for the reported performance issue, whereupon another maintenance operation in the maintenance task template may be executed in a similar manner.

In a further example, the operation of selecting a maintenance task template for resolving the performance issue select template, is performed based on a similarity between the extracted one or more issue features, and corresponding issue features in the maintenance task templates in the database. In so doing, an optimal maintenance task template may be selected. Further factors may also be used to guide the selection of the maintenance task template, such as for example the time within which the performance issue is expected to be resolved, the availability of replacement components that are defined in the maintenance task template, and the availability of service engineers with an appropriate training to perform a maintenance operations defined in the maintenance task template.

In a further example, the maintenance task template includes a plurality of related maintenance operations for resolving the issue, and each maintenance operation comprises a requirement for completing said maintenance operation. The method further includes:

- populating the maintenance task template by providing a maintenance operation of the maintenance task template to a question answering, QA, algorithm, the QA algorithm being trained to generate a Boolean indication for indicating whether the requirement of the maintenance operation can be met;
- obtaining a prediction result from the QA algorithm, the prediction result comprising one or more Boolean indications for the maintenance operation; and
- categorizing the maintenance operation into one of a plurality of categories based on the prediction result.

In so doing, an effective technique is provided for assessing the maintenance operations of a maintenance task template in order to predict the likelihood of the maintenance operations being successfully completed by the user. The maintenance operations of the maintenance task template may then be categorized according to the prediction results so that they can then be addressed accordingly. Put another way, this provides an algorithmic approach for effective assessment of a performance issue by identifying what is missing from the maintenance operations, and filling in the gaps in an efficient manner. When filled, each maintenance operation may include a clear 'how to' manual, including for example the required expertise description, tools, spare parts and the like needed for completing the maintenance operation. In other words, this may assist in optimizing the above-mentioned resources between the categories of maintenance operations, thereby ensuring an efficient and swift resolution to the performance issue.

In an example, the QA algorithm is a natural language processing algorithm, and wherein the QA algorithm is adapted to generate the Boolean indication using unstructured information sources.

In this way, the Boolean indication may be generated based on any available information source.

In a further example, the QA algorithm is adapted to generate the Boolean indication using unstructured information sources based on a query aware vector.

In this way, the Boolean indication may be generated with greater accuracy and computational efficiency.

In an example, the QA algorithm is a natural language processing algorithm, and wherein the QA algorithm is adapted to generate the Boolean indication using structured information sources.

In this way, the Boolean indication may be generated based on any available prepared information source.

In an example, the QA algorithm is further adapted to:

identify a user assigned to complete the maintenance operation;
compare an expertise of the user with the requirement for completing the maintenance operation; and
adjust the Boolean indication based on the comparison.

In this way, it is possible to determine whether a user assigned to resolve a given performance issue is capable of doing so based on a record of said user's experience.

In a further example, if the expertise of the user in not compatible with the requirement for completing the maintenance operation, the QA algorithm is further adapted to:

identify at least one other user with expertise that is compatible with the requirement for completing the maintenance operation; and
generate a request for to be provided to the at least one other user, wherein the request comprises a request for the at least one other user's input in completing the maintenance operation.

In this way, a user with the requisite amount of experience for ensuring that a performance issue is resolved to a given standard may be identified to aid the originally assigned user.

In an example, the method further comprises:

identifying a context of the maintenance operation within the maintenance task template; and
assigning a priority rating to the maintenance operation based on the identified context.

In this way, it may be possible to assign a higher priority to maintenance operations that are more critical to the solution of the performance issue so that these maintenance operations are addressed before less critical maintenance operations.

In an example, the requirement for completing the maintenance operation comprises one or more of:

an instruction for completing the maintenance operation;
a required experience level of a user;
a tool for completing the maintenance operation;
an available second user to assist in completing the maintenance operation.

According to an example in accordance with an aspect of the present disclosure, there is provided a computer-implemented method of training a machine learning algorithm to process a maintenance task template for resolving a performance issue. The maintenance task template includes a plurality of related maintenance operations for resolving the performance issue, and each maintenance operation comprises a requirement for completing said maintenance operation. The method includes:

- receiving: i) a training unstructured information source comprising a training query and a training text comprising an answer to the training query; and ii) a training Boolean indication; and
- training the machine learning algorithm based on the training unstructured information source as a training input for the machine learning algorithm and the training Boolean indication as the training output for the machine learning algorithm.

In this way, a machine learning algorithm for processing a maintenance task template for resolving a performance issue may be provided in an accurate and efficient manner.

In an example, training the machine learning algorithm comprises:

feeding the training unstructured information source to an embedding layer, wherein the embedding layer is adapted to represent words in a vector space;
converting, by way of the embedding layer, the training query into a query vector and the training text into an answer vector

In an example, training the machine learning algorithm further comprises:
applying an attention function to the query vector and the answer vector in order to generate a query aware vector

In this way, unstructured data may be utilized to accurately and efficiently train the machine learning algorithm to generate the Boolean indicator.

In an example, the method further comprises:

receiving: iii) a training expertise record of a user; and iv) a training Boolean indication adjustment; and
training the machine learning algorithm based on the training expertise record of a user as a training input for the machine learning algorithm and the training Boolean indication adjustment as the training output for the machine learning algorithm.

In this way, the machine learning algorithm may be trained to adjust the Boolean indication based on an amount of experience an assigned user has for resolving the given performance issue.

According to examples in accordance with an aspect of the present disclosure, there is provided a computer program product comprising computer program code means which, when executed on a computing device having a processing system, cause the processing system to perform the methods described above.

According to an example in accordance with an aspect of the present disclosure, there is provided a system for performing a maintenance operation on a medical imaging system in response to a performance issue. The system includes a processor adapted to:

- receive input data representing the performance issue;
- extract one or more issue features from the input data, the extracted issue features including one or more of: a type of process executed on or by the medical imaging system prior to a time of the performance issue, an identification of a subcomponent installed in the medical imaging system, an amount of wear of a component of the imaging system, sensor data from the medical imaging system, a history of previous maintenance operations or software updates performed on the medical imaging system, and an expected time within which the performance issue must be resolved;
- select, based on the extracted one or more issue features, a maintenance task template for resolving the performance issue, wherein the maintenance task template is selected from a database of maintenance task templates, each maintenance task template defining one or more maintenance operations to be performed on the medical imaging system to resolve performance issues; and
- execute the one or more maintenance operations defined in the selected maintenance task template on the medical imaging system, in order to resolve the medical imaging system performance issue.

According to an example in accordance with an aspect of the present disclosure, there is provided a system for training a machine learning algorithm to process a maintenance task template for resolving a performance issue. The maintenance task template includes a plurality of related maintenance operations for resolving the performance issue, and each maintenance operation comprises a requirement for completing said maintenance operation. The system includes a processor adapted to:

- receive: i) a training unstructured information source comprising a training query and a training text comprising an answer to the training query; and ii) a training Boolean indication; and
- train the machine learning algorithm based on the training unstructured information source as a training input for the machine learning algorithm and the training Boolean indication as the training output for the machine learning algorithm.

These and other aspects of the present disclosure will be apparent from and elucidated with reference to the example(s) described hereinafter.

BRIEF DESCRIPTION OF THE DRAWINGS

In order to facilitate an improved understanding of the present disclosure, and to show more clearly how it may be carried into effect, reference is now made, by way of example only, to the accompanying drawings, in which:

Fig. 1 shows a method for processing a maintenance task template according to an aspect of the present disclosure;
Fig. 2 shows an example of a QA algorithm for generating the Boolean indication using structured information sources;
Fig. 3 shows an example of training a QA algorithm for generating the Boolean indication using unstructured information sources; and
Fig. 4 shows an example of a computer within which one or more parts of an example may be employed.

DETAILED DESCRIPTION OF EMBODIMENTS

The present disclosure will be described with reference to the Figures.

It should be understood that the detailed description and specific examples of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The present disclosure provides a computer-implemented method of performing a maintenance operation on a medical imaging system in response to a performance issue. The method comprises:

- receiving input data representing the performance issue;
- extracting one or more issue features from the input data, the extracted issue features including one or more of: a type of process executed on or by the medical imaging system prior to a time of the performance issue, an identification of a subcomponent installed in the medical imaging system, an amount of wear of a component of the imaging system, sensor data from the medical imaging system, a history of previous maintenance operations or software updates performed on the medical imaging system, and an expected time within which the performance issue must be resolved;
- selecting, based on the extracted one or more issue features, a maintenance task template for resolving the performance issue, wherein the maintenance task template is selected from a database of maintenance task templates, each maintenance task template defining one or more maintenance operations to be performed on the medical imaging system to resolve performance issues; and
- executing the one or more maintenance operations defined in the selected maintenance task template on the medical imaging system, in order to resolve the medical imaging system performance issue.

By way of some examples, the medical imaging system may for example be an X-ray, a computed tomography, a positron emission tomography "PET", a single photon emission computed tomography "SPECT", or a magnetic resonance "MR" imaging system. Performance issues may occur in such imaging systems in the form of a reduction in the image quality of images generated by the imaging system, an increase in the time required to complete various operations such as the reconstruction of images or to position moving parts of the imaging system. A reduction in image quality may for example be present in the form of a reduction in signal so noise ratio, image blurring, or the presence of artifacts in medical images, and so forth.

The input data representing the performance issue may for example include log file data and/or a technical description of the performance issue. Log file data is routinely generated by a medical imaging system, and includes information such the values of parameters used to acquire and reconstruct medical images, sensor data representing e.g. a temperature, vibration, and a motion of a component of the imaging system during image acquisition, a power supply voltage, a power supply current, and environmental data representing e.g. a temperature, humidity, and so forth of an environment in which the imaging system is operated. In general, the log file data may include information representing: a type of process executed on or by the medical imaging system prior to a time of the performance issue, an identification of components installed in the medical imaging system, sensor data representing an amount of wear of a component of the imaging system, sensor data from the medical imaging system representing e.g. a temperature, or a motion of a component of the imaging system, a history of previous maintenance operations or software updates performed on the medical imaging system, and so forth.

As mentioned above, the technical description of the performance issue may be provided by an operator of the medical imaging system. The technical description may include similar information such as that included in the log file data, as described above. Sometimes, the technical description is provided at a high level by the operator reporting the performance issue, and may only include information such as 'imaging system with model number xyz is generating images have high levels of noise'. The operator may also provide a request to resolve the performance issue within a particular time. The time within which the performance issue is expected to be resolved may have been previously agreed in a contractual agreement between a medical facility in which the medical imaging system is located, and a servicing facility. Upon receipt of such a technical description, the service engineer may ask the operator to provide further information on the technical description by requesting details, such as the process that was being executed by the medical imaging system before the time of the performance issue being observed. The service engineer may also investigate the log data for more information.

The input data may in general be received via any form of communication, including verbally, e.g. via a telephone, via a messaging application such as email, or via the Internet or the Cloud. An analysis may be performed on the input data, for example on the technical description, in order to convert the technical description into a computer-readable form. A natural language processing technique such as voice to text, may for example be performed on the received input data such that it may be processed by a computer.

One or more issue features are then extracted from the input data. The extracted issue features may include one or more of: a type of process executed on or by the medical imaging system prior to a time of the performance issue, an identification of a subcomponent installed in the medical imaging system, an amount of wear of a component of the imaging system, sensor data from the medical imaging system, a history of previous maintenance operations or software updates performed on the medical imaging system, and an expected time within which the performance issue must be resolved. The issue features may be extracted using a natural language processing "NLP" technique, for example.

A maintenance task template for resolving the performance issue is then selected based on the extracted one or more issue features. The maintenance task template is selected from a database of maintenance task templates.

Each maintenance task template defines one or more maintenance operations to be performed on the medical imaging system to resolve performance issues. The maintenance task template may also be selected based on one or more of an identification of a type, or a model number of the medical imaging system; an inventory of components available to resolve the maintenance issue, an availability of diagnostic tools for identifying a root cause of the performance issue, and a type of maintenance contract associated with the medical imaging system. The maintenance contract may define an expected time within which the performance issue must be resolved.

After the maintenance task template has been selected, the one or more maintenance operations defined in the selected maintenance task template are executed on the medical imaging system in order to resolve the medical imaging system performance issue. Examples of maintenance operations that may be executed in this operation include performing a self-update of software, performing diagnostic tests on hardware components, such as rotating a gantry of a CT imaging system and measuring an amount of resulting vibration, the reading-out of sensor data, making adjustments to parameters of the imaging system such as for example operating voltages of an X-ray tube, calibrating imaging system parameters and determining their impact on noise in images generated by the medical imaging system.

Thus, the use of a maintenance task template as described in the above method provides an efficient means of resolving the performance issue. Since the maintenance task template is selected based on the extracted issue features, an optimal appropriate maintenance task template may be selected for resolving the performance issue. Moreover, the method permits the performance issues that have similar issue features to be resolved in a similar manner. As a consequence, the maintenance operation may be resolved within a predictable timeframe, which helps to minimize the impact of the performance issue on clinical workflow.

In some examples, the operation of executing the one or more maintenance operations comprises performing a diagnostic test on the medical imaging system, and adjusting one or more operating parameters of the medical imaging system in response to a result of the diagnostic test. Examples of diagnostic tests that may be performed in this operation include measuring the voltage and exposure time of an X-ray tube of an X-ray or CT imaging system, and measuring a voltage at which arcing occurs in the tube. If the peak energy of the output beam is not same as the set kVp, image quality may be degraded. Variations in the exposure time of an X-ray tube can result in dose variation and consequently the diagnostic value of a medical image. The operating parameters of the medical imaging system that may be adjusted in response to these diagnostic tests include the operating voltage, and the exposure time. For instance, the operating voltage may be adjusted to a value within specified limits, or reduced to a level at which the probability of arcing is reduced. Another example of a diagnostic test that may be performed in this operation is a determination of a motor speed. The motor may control a speed of CT gantry rotation, X-ray C-arm movement, or a speed of movement of a patient table, for example. In response to a determination of the speed, various settings may be adjusted such that the speed is within specified limits. Another example of a diagnostic test that may be performed in this operation is to perform a comparison of the image quality of an image generated by the medical imaging system with a reference image. The reference image may be generated by a phantom and stored in a database. The comparison may involve a determination of the sharpness, or noise, in the image with respect to the reference image. In response to the comparison, parameters of the medical imaging system may be adjusted, such as for example a position of an X-ray source respective the X-ray detector, or the values of image reconstruction parameters.

The operation of selecting a maintenance task template for resolving the performance issue select template may be performed based in various ways. In one example, the maintenance task template may be selected based on a similarity between the extracted one or more issue features, and corresponding issue features in the maintenance task templates in the database. By way of an example, the issue features may be represented as text in the received input data. In the event that the issue features are received as voice data, the voice data may be extracted by converting the voice data to text using a voice-to-text technique. Other NLP techniques may also be used to extract the issue features from voice and/or text data. The issue features extracted from the input data and the issue features in the template are then transformed to vector space e.g. $V_{issues}$ and $V_{template}$. Word embedding is used to transform text features to vector space. Word embedding represents words in a vector space where similar words are mapped near each other based on the context of usage. This can be performed using a word-embedding model that is trained using the knowledge base/ corpus of the specific maintenance domain. Next, a vector similarity function, such as cosine similarity, is applied to the vectors in order to calculate a similarity score from these two vectors. A threshold may then be used to determine which of the templates to select:

$$f_{Similarity}\left(V_{issues}, V_{template}\right) > threshold_{Similarity} \qquad \text{(Equation 1)}$$

As an example, a user may report the performance issue "over exposure has been observed in the X-ray images". One root cause of this issue could be a malfunction of the exposure controller. A maintenance task template may exist for this issue. In this example, the text/ words "exposure", and "image" extracted from the input data will have a high similarity score with features in the maintenance task template for resolving a malfunction of the exposure controller.

Thus, this template will be selected as the maintenance task template for resolving the performance issue. The maintenance task template may also be selected based on an expected time to complete the one or more maintenance operations defined in the maintenance task template. For example if the expected time for completion is relatively short, a template where many tasks are completed in parallel may be preferred, with the risk that some tasks may in time become obsolete. However if the expected time for completion is relatively long, a template where few - if any - tasks are completed in parallel may be preferred. In such a manner the risk that some tasks may be carried out unnecessarily is reduced compared to the parallel template option.

In one example, the maintenance task template comprises a plurality of related maintenance operations for resolving the performance issue, and each maintenance operation comprises a requirement for completing said maintenance operation. In this example, the method comprises: populating the maintenance task template by providing a maintenance operation of the maintenance task template to a question answering, QA, algorithm, the QA algorithm being trained to generate a Boolean indication for indicating whether the requirement of the maintenance operation can be met; obtaining a prediction result from the QA algorithm, the prediction result comprising one or more Boolean indications for the maintenance operation; and categorizing the maintenance operation into one of a plurality of categories based on the prediction result.

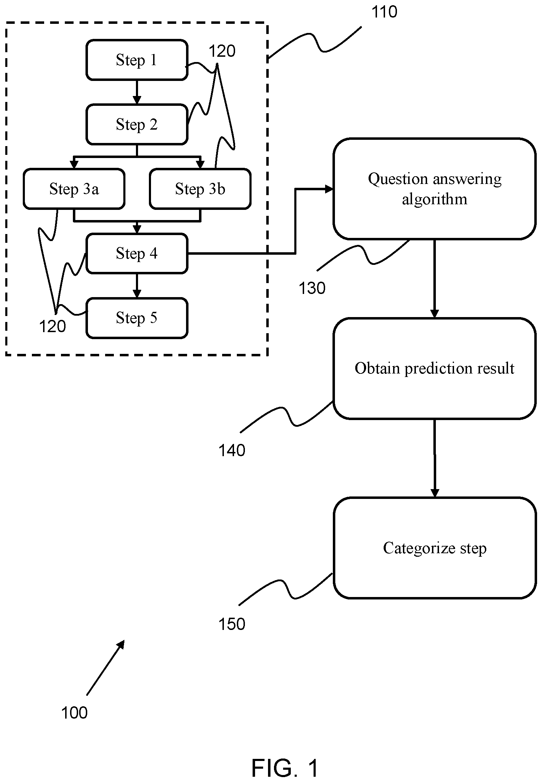

Fig. 1 shows a method 100 for processing a maintenance task template 110 according to an aspect of the present disclosure. The maintenance task template comprises a plurality of related maintenance operations 120 for resolving the performance issue and each maintenance operation comprises a requirement for completing said maintenance operation.

The requirement for completing the maintenance operation may comprise one or more of: an instruction for completing the maintenance operation; a required experience level of a user; a tool for completing the maintenance operation; and an available second user to assist in completing the maintenance operation.

A maintenance operation 120 of the maintenance task template 110 is provided to a question answering, QA, algorithm 130. The QA algorithm is trained to generate a Boolean indication for indicating whether the requirement of the maintenance operation can be met. The training of the QA algorithm is described in further detail below.

In some examples, the method may further comprise a step of identifying a context of the maintenance operation within the maintenance task template. A priority rating may then be assigned to the step based on the identified context. In other words, the method may include the step of determining whether a maintenance operation is critical or non-critical to the solution of the performance issue. Further, the priority rating assigned to the maintenance operation may be based on how time sensitive the maintenance operation is within the context of the performance issue. For example, if a maintenance operation is not time sensitive, the priority rating may be lower than for a maintenance operation that is highly time sensitive.

In operation 140, a prediction result comprising one or more Boolean indications for the maintenance operation is obtained from the QA algorithm. The QA algorithm is discussed in further detail below.

In operation 150, the maintenance operation is categorized into one of a plurality of categories based on the prediction result. For example, the plurality of categories may include one or more of: all requirements for solution met; some requirements for solution met; no requirements for solution met; critical; non-critical; time sensitive; not time sensitive; or any combination thereof.

For example, there may be a slots, or maintenance operations, where not all of the requirements are met, but which are less critical to resolve than other slots. Such slots may, for example, relate to cases where work can still be performed but perhaps less efficiently, for example when the assigned user does not have sufficient experience and would require more time as they would need to refer to instruction manuals, or where there is a known delay, for example in the availability of a spare part.

The methods described herein may provide an algorithmic approach for the effective assessment of a maintenance task template, identifying maintenance operations that are missing certain requirements and, in some example, filling in the missing requirements where possible in an efficient manner. When filled, each maintenance operation may contain a clear manual for performing the maintenance operation, and may include the required expertise description, tools, spare parts and the like required for completing the work.

There is now provided a worked example of the methods described with reference to Fig. 1. A slot, or maintenance operation, in the maintenance task template may be considered as being a separable block of work that needs to be performed in order to resolve the performance issue. As described above, slots have different sets of requirements, or properties.

In this example, a simple slot may be the removal of an MRI bore cover. Such a maintenance operation may have the following requirements to perform the work: illustrated instructions of which screws to remove and in which order; a user with an experience score of performing this particular task above a predetermined threshold value; a minimum set of tools; and an availability of a second user to hold the cover as the first user removes the screws. Each requirement may be accompanied by a Boolean indicator (true/false) to indicate whether the requirement can be met based on currently available resources.

The number of requirements that receive a true Boolean indicator and the number of requirements that receive a false Boolean indicator for each maintenance operation will be used to generate the prediction result and categorize the maintenance operation.

The QA algorithm may be a natural language processing algorithm and may be adapted to generate the Boolean indication using unstructured information sources. The training of the QA algorithm and its utilization with unstructured information sources is discussed further below.

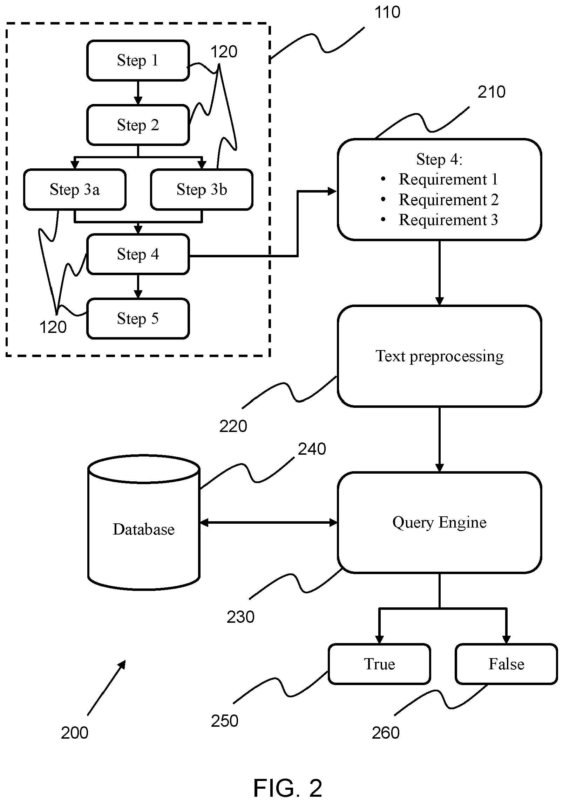

The QA algorithm may also be adapted to generate the Boolean indication using structured information sources. Fig. 2 shows an example 200 of a QA algorithm for generating the Boolean indication using structured information sources.

In the example shown in Fig. 2, the requirements 210 of a slot may be fed as query inputs to the QA algorithm and in operation 220 preprocessing of the query text may be performed. The text preprocessing may involve tokenization of the query sentences and stemming of the words to their base form. Then Parts of Speech and Named Entity recognition of the words may be performed. A query engine 230 may then receive the preprocessed text and search in a structured database 240 for answers. The database, or databases, may consist of schemas for inventory lists, user experience profiles and the like. Based on the search result of the database, the query engine may provide a Boolean answer of true 250 or false 260 for each of the requirements of the maintenance operation.

Thus the QA algorithm may be of two types. One type may infer the answer of the requirements of the slot from unstructured information sources such as service manuals, technical documents and the like, as described below; whereas the other type may provide the answer using a deterministic approach by consulting structured databases that contain inventory information, previous historical records of performance issue resolving, availability of users and the like.

As mentioned above, one of the requirements for resolving a maintenance operation of the maintenance task template may be a minimum expertise of the user in performing the required work. In this case, the QA algorithm may be further adapted to: identify a user assigned to complete the maintenance operation; compare an expertise of the user with the requirement for completing the maintenance operation; and adjust the Boolean indication based on the comparison.

The expertise of a user may be measured according to any suitable metric, such as: a number of times the user has performed a given task; a length of time the user has been working; a qualification of the user; and the like. The adjustment of the Boolean indication may include providing a true Boolean response if the user meets the predetermined threshold of experience or providing a false Boolean response if the user does not meet the predetermined threshold of experience.

In addition, if the expertise of the user is not compatible with the requirement for completing the maintenance operation, or if the user does not meet the predetermined threshold of experience, the QA algorithm may be further adapted to: identify at least one other user with expertise that is compatible with the requirement for completing the maintenance operation; and generate a request for to be provided to the at least one other user, wherein the request comprises a request for the at least one other user's input in completing the maintenance operation.

In other words, if the QA algorithm identifies a maintenance operation which requires a greater experience level than possessed by the assigned user, the input may be crowd sourced from at least one other user with the requisite experience level.

For example, when the user working on the maintenance operation lacks experience, which may be judged from their documented experience, the QA algorithm may initiate and distribute a call for help reaching out to users within the network who have the requisite experience for completing the maintenance operation, such as a sufficient amount of successfully resolved cases on the subject in hand. Further, the QA algorithm may take into account the availability of the support network in order to ensure a swift response to the request.

In the case where multiple other users answer the request, the answers may be analyzed and the assigned user provided with a single answer that is either chosen by majority voting or automatically generated from a combination of the provided answers. The automatically generated combination of answers may be adjusted so that the user receives a concise, yet complete picture of the proposed solution of the performance issue at hand.

Each slot of the maintenance task template may possess requirements that can be automatically filled, for example, by providing the relevant instruction for performing a task from a database. When attempting to fill a slot, the performance issue description text, user manuals and other sources of information may be scanned to locate matching field names and their corresponding values when available. Those values can be parameter values, lists of needed parts, and the like. The information may then be provided to the slot as required.

The present disclosure further provides a computer-implemented method of training a machine learning algorithm to process a maintenance task template, the maintenance task template for resolving a performance issue. The maintenance task template comprises a plurality of related maintenance operations for resolving the performance issue, and each maintenance operation comprises a requirement for completing said maintenance operation. The method comprises: receiving: i) a training unstructured information source comprising a training query and a training text comprising an answer to the training query; and ii) a training Boolean indication; and training the machine learning algorithm based on the training unstructured information source as a training input for the machine learning algorithm and the training Boolean indication as the training output for the machine learning algorithm.

A machine-learning algorithm is any self-training algorithm that processes input data in order to produce or predict output data. Here, the input data comprises training unstructured information and the output data comprises training Boolean indications.

Suitable machine-learning algorithms for being employed in the present disclosure will be apparent to the skilled person. Examples of suitable machine-learning algorithms include decision tree algorithms and artificial neural networks. Other machine-learning algorithms such as logistic regression, support vector machines or Naive Bayesian models are suitable alternatives.

The structure of an artificial neural network (or, simply, neural network) is inspired by the human brain. Neural networks are comprised of layers, each layer comprising a plurality of neurons. Each neuron comprises a mathematical operation. In particular, each neuron may comprise a different weighted combination of a single type of transformation (e.g. the same type of transformation, sigmoid etc. but with different weightings). In the process of processing input data, the mathematical operation of each neuron is performed on the input data to produce a numerical output, and the outputs of each layer in the neural network are fed into the next layer sequentially. The final layer provides the output.

Methods of training a machine-learning algorithm are well-known. Typically, such methods comprise obtaining a training dataset, comprising training input data entries and corresponding training output data entries. An initialized machine-learning algorithm is applied to each input data entry to generate predicted output data entries. An error between the predicted output data entries and corresponding training output data entries is used to modify the machine-learning algorithm. This process can be repeated until the error converges, and the predicted output data entries are sufficiently similar (e.g. $\pm 1\%$) to the training output data entries. This is commonly known as a supervised learning technique.

For example, where the machine-learning algorithm is formed from a neural network, (weightings of) the mathematical operation of each neuron may be modified until the error converges. Known methods of modifying a neural network include gradient descent, backpropagation algorithms and so on.

The training input data entries correspond to example training unstructured information. The training output data entries correspond to training Boolean indications.

The QA algorithm using unstructured data sources has two primary objectives, the first of which is to model the queries and the corresponding answers contained within the information source and the second of which is to tag them with a Boolean output.

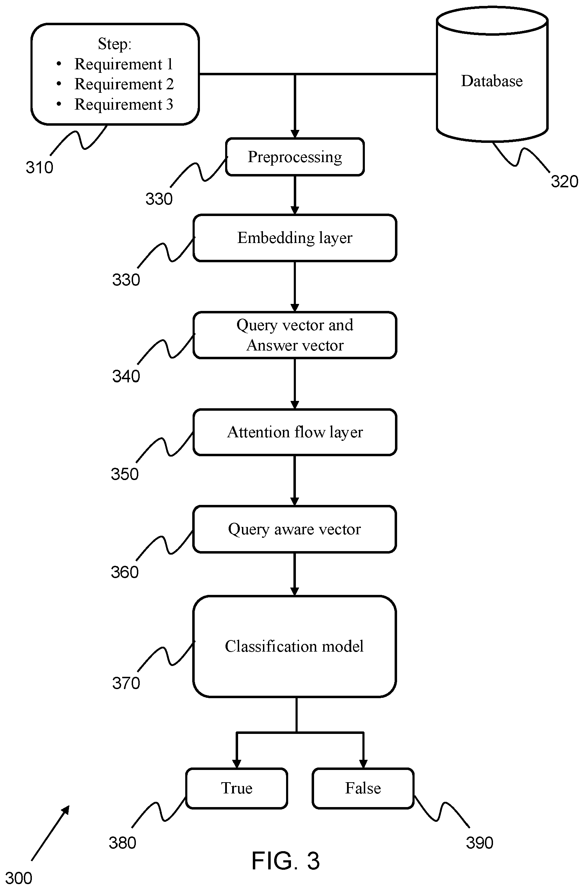

Fig. 3 shows an example 300 of training a QA algorithm for generate the Boolean indication using structured information sources

The QA algorithm may be trained using slot attributes 310 as queries, paragraphs containing the answers, obtained from a database 320, and Boolean classifications for those answers. The training dataset may be prepared as a combination of a question, a paragraph and a Boolean answer, which is either true of false. The performance issue definition may be: for a given query and paragraph, predict the Boolean answer class.

The training data may then be preprocessed 330 and fed to the embedding layer 330. The embedding layer represents words in a vector space where similar words may be mapped near each other based on the context of their use. A query vector and an answer vector may be generated 340 for the query and the paragraph respectively.

The query and answer vectors may then be provided as inputs to an attention flow layer 350, where an attention function may be applied to the vectors to generate a query aware vector 360 representation of each word.

The query aware vector representation of the query and the answer paragraph may then be fed to a CNN based classification model 370 as an input. Such a model may consist of multiple convolution layers, max pooling, drop out and fully connected layers and a Sigmoid/ Softmax layer to classify the input as one of the Boolean answer classes, true 380 or false 390.

In an alternative implementation, the QA algorithm may be trained using a Bidirectional Encoder Representations from Transformers (BERT) technique, which is a transformer-based machine learning technique for natural language processing. In this case, the QA algorithm may be trained on unstructured information source using a BERT model, which may be fine-tuned on a service domain in two stages.

In the first stage, the BERT model may be pre-trained with service domain text using masked language modelling to generate appropriate embedding for the sentences/words in the service domain. In the second stage, questions and answers may be fed into the BERT model for fine-tuning it for the question answering task. The BERT output may then be used as an input to the above mentioned CNN based classification model to classify the input as Boolean answer classes.

In a further example, the QA algorithm may be trained training the machine learning algorithm based on the training expertise record of a user as a training input for the machine learning algorithm and the training Boolean indication adjustment as the training output for the machine learning algorithm.

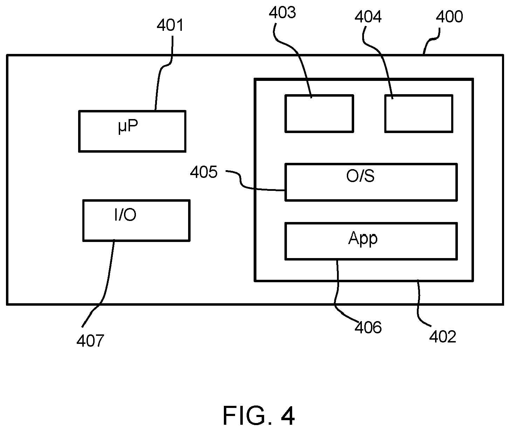

Fig. 4 illustrates an example of a computer 400 within which one or more parts of an example may be employed. Various operations discussed above may utilize the capabilities of the computer 400. For example, one or more parts of a system for processing a maintenance task template may be incorporated in any element, module, application, and/or component discussed herein. In this regard, it is to be understood that system functional blocks can run on a single computer or may be distributed over several computers and locations (e.g. connected via internet).

The computer 400 includes, but is not limited to, PCs, workstations, laptops, PDAs, palm devices, servers, storages, and the like. Generally, in terms of hardware architecture, the computer 400 may include one or more processors 401, memory 402, and one or more I/O devices 407 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 401 is a hardware device for executing software that can be stored in the memory 402. The processor 401 can be virtually any custom made or commercially available processor, a central processing unit "CPU", a digital signal processor "DSP", or an auxiliary processor among several processors associated with the computer 400, and the processor 401 may be a semiconductor based microprocessor (in the form of a microchip) or a microprocessor.

The memory 402 can include any one or combination of volatile memory elements (e.g., random access memory "RAM", such as dynamic random access memory "DRAM", static random access memory "SRAM", etc.) and non-volatile memory elements (e.g., ROM, erasable programmable read only memory "EPROM", electronically erasable programmable read only memory "EEPROM", programmable read only memory "PROM", tape, compact disc read only memory "CD-ROM", disk, diskette, cartridge, cassette or the like, etc.). Moreover, the memory 402 may incorporate electronic, magnetic, optical, and/or other types of storage media. Note that the memory 402 can have a distributed architecture, where various components are situated remote from one another, but can be accessed by the processor 401.

The software in the memory 402 may include one or more separate programs, each of which comprises an ordered listing of executable instructions for implementing logical functions. The software in the memory 402 includes a suitable operating system "O/S" 405, compiler 404, source code 403, and one or more applications 406 in accordance with exemplary examples. As illustrated, the application 406 comprises numerous functional components for implementing the features and operations of the exemplary examples. The application 406 of the computer 400 may represent various applications, computational units, logic, functional units, processes, operations, virtual entities, and/or modules in accordance with exemplary examples, but the application 406 is not meant to be a limitation.

The operating system 405 controls the execution of other computer programs, and provides scheduling, input-output control, file and data management, memory management, and communication control and related services. It is contemplated by the inventors that the application 406 for implementing exemplary examples may be applicable on all commercially available operating systems.

Application 406 may be a source program, executable program (object code), script, or any other entity comprising a set of instructions to be performed. When a source program, then the program is usually translated via a compiler (such as the compiler 404), assembler, interpreter, or the like, which may or may not be included within the memory 402, so as to operate properly in connection with the O/S 405. Furthermore, the application 406 can be written as an object oriented programming language, which has classes of data and methods, or a procedure programming language, which has routines, subroutines, and/or functions, for example but not limited to, C, C++, C#, Pascal, BASIC, API calls, HTML, XHTML, XML, ASP scripts, JavaScript, FORTRAN, COBOL, Perl, Java, ADA, .NET, and the like.

The I/O devices 407 may include input devices such as, for example but not limited to, a mouse, keyboard, scanner, microphone, camera, etc. Furthermore, the I/O devices 407 may also include output devices, for example but not limited to a printer, display, etc. Finally, the I/O devices 407 may further include devices that communicate both inputs and outputs, for instance but not limited to, a NIC or modulator/demodulator (for accessing remote devices, other files, devices, systems, or a network), a radio frequency "RF" or other transceiver, a telephonic interface, a bridge, a router, etc. The I/O devices 407 also include components for communicating over various networks, such as the Internet or intranet.

If the computer 400 is a PC, workstation, intelligent device or the like, the software in the memory 402 may further include a basic input output system "BIOS" (omitted for simplicity). The BIOS is a set of essential software routines that initialize and test hardware at startup, start the O/S 405, and support the transfer of data among the hardware devices. The BIOS is stored in some type of read-only-memory, such as ROM, PROM, EPROM, EEPROM or the like, so that the BIOS can be executed when the computer 400 is activated.

When the computer 400 is in operation, the processor 401 is configured to execute software stored within the memory 402, to communicate data to and from the memory 402, and to generally control operations of the computer 400 pursuant to the software. The application 406 and the O/S 405 are read, in whole or in part, by the processor 401, perhaps buffered within the processor 401, and then executed.

When the application 406 is implemented in software it should be noted that the application 406 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 406 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

Variations to the disclosed examples can be understood and effected by those skilled in the art, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". If the term "arrangement" is used in the claims or description, it is noted the term "arrangement" is intended to be equivalent to the term "system", and vice versa.

Any reference signs in the claims should not be construed as limiting the scope.

## Claims

1. A computer-implemented method of performing a maintenance operation on a medical imaging system in response to a performance issue, wherein the method comprises:

   receiving input data representing the performance issue;
   extracting one or more issue features from the input data, the extracted issue features including one or more of: a type of process executed on or by the medical imaging system prior to a time of the performance issue, an identification of a subcomponent installed in the medical imaging system, an amount of wear of a component of the imaging system, sensor data from the medical imaging system, a history of previous maintenance operations or software updates performed on the medical imaging system, and an expected time within which the performance issue must be resolved;
   selecting, based on the extracted one or more issue features, a maintenance task template for resolving the performance issue, wherein the maintenance task template is selected from a database of maintenance task templates, each maintenance task template defining one or more maintenance operations to be performed on the medical imaging system to resolve performance issues; and
   executing the one or more maintenance operations defined in the selected maintenance task template on the medical imaging system, in order to resolve the medical imaging system performance issue.

2. The computer-implemented method according to claim 1, wherein the executing the one or more maintenance operations comprises:

   performing a diagnostic test on the medical imaging system; and
   adjusting one or more operating parameters of the medical imaging system in response to a result of the diagnostic test.

3. The computer-implemented method according to claim 1 or claim 2, wherein the selecting a maintenance task template for resolving the performance issue select template, is performed based on a similarity between the extracted one or more issue features, and corresponding issue features in the maintenance task templates in the database.

4. The computer-implemented method according to claim 1, wherein the maintenance task template comprises a plurality of related maintenance operations for resolving the performance issue, wherein each maintenance operation comprises a requirement for completing said maintenance operation, wherein the method further comprises:

   populating the maintenance task template by providing a maintenance operation of the maintenance task template to a question answering, QA, algorithm, the QA algorithm being trained to generate a Boolean indication for indicating whether the requirement of the maintenance operation can be met;

obtaining a prediction result from the QA algorithm, the prediction result comprising one or more Boolean indications for the maintenance operation; and

categorizing the maintenance operation into one of a plurality of categories based on the prediction result.

5. The computer-implemented method as claimed in claim 4, wherein the QA algorithm is a natural language processing algorithm, and wherein the QA algorithm is adapted to generate the Boolean indication using unstructured information sources.

6. The computer-implemented method as claimed in claim 2, wherein the QA algorithm is adapted to generate the Boolean indication using unstructured information sources based on a query aware vector.

7. The computer-implemented method as claimed in any of claims 1 to 3, wherein the QA algorithm is a natural language processing algorithm, and wherein the QA algorithm is adapted to generate the Boolean indication using structured information sources.

8. The computer-implemented method as claimed in any of claims 1 to 4, wherein the QA algorithm is further adapted to:

identify a user assigned to complete the maintenance operation;
compare an expertise of the user with the requirement for completing the maintenance operation; and
adjust the Boolean indication based on the comparison

9. The computer-implemented method as claimed in claim 5, wherein, if the expertise of the user in not compatible with the requirement for completing the maintenance operation, the QA algorithm is further adapted to:

identify at least one other user with expertise that is compatible with the requirement for completing the maintenance operation; and
generate a request for to be provided to the at least one other user, wherein the request comprises a request for the at least one other user's input in completing the maintenance operation.

10. The computer-implemented method as claimed in any of claims 1 to 6, wherein the method further comprises:

identifying a context of the maintenance operation within the maintenance task template; and
assigning a priority rating to the maintenance operation based on the identified context.

11. The computer-implemented method as claimed in any of claims 1 to 7, wherein the requirement for completing the maintenance operation comprises one or more of:

an instruction for completing the maintenance operation;
a required experience level of a user;
a tool for completing the maintenance operation;
an available second user to assist in completing the maintenance operation.

12. A computer-implemented method of training a machine learning algorithm to process a maintenance task template for resolving a performance issue, the maintenance task template comprising a plurality of related maintenance operations for resolving the performance issue, wherein each maintenance operation comprises a requirement for completing said maintenance operation, the method comprising:

receiving: i) a training unstructured information source comprising a training query and a training text comprising an answer to the training query; and ii) a training Boolean indication; and
training the machine learning algorithm based on the training unstructured information source as a training input for the machine learning algorithm and the training Boolean indication as the training output for the machine learning algorithm.

13. A computer program product comprising computer program code means which, when executed on a computing device having a processing system, cause the processing system to perform the method according to any of claims 1 to 12.

14. A system for performing a maintenance operation on a medical imaging system in response to a performance issue, wherein the system comprises a processor adapted to:

receive input data representing the performance issue;

extract one or more issue features from the input data, the extracted issue features including one or more of: a type of process executed on or by the medical imaging system prior to a time of the performance issue, an identification of a subcomponent installed in the medical imaging system, an amount of wear of a component of the imaging system, sensor data from the medical imaging system, a history of previous maintenance operations or software updates performed on the medical imaging system, and an expected time within which the performance issue must be resolved;

select, based on the extracted one or more issue features, a maintenance task template for resolving the performance issue, wherein the maintenance task template is selected from a database of maintenance task templates, each maintenance task template defining one or more maintenance operations to be performed on the medical imaging system to resolve performance issues; and

execute the one or more maintenance operations defined in the selected maintenance task template on the medical imaging system, in order to resolve the medical imaging system performance issue.

15. A system for training a machine learning algorithm to process a maintenance task template for resolving a performance issue, the maintenance task template comprising a plurality of related maintenance operations for resolving the performance issue, wherein each maintenance operation comprises a requirement for completing said maintenance operation, wherein the system comprises a processor adapted to:

receive: i) a training unstructured information source comprising a training query and a training text comprising an answer to the training query; and ii) a training Boolean indication; and

train the machine learning algorithm based on the training unstructured information source as a training input for the machine learning algorithm and the training Boolean indication as the training output for the machine learning algorithm.

FIG. 1

FIG. 2

FIG. 3

FIG. 4

Europäisches
Patentamt

European
Patent Office

Office européen
des brevets

# EUROPEAN SEARCH REPORT

Application Number

EP 22 15 4269

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2004/093184 A1 (HENDRICKSON DAVID [US]) 13 May 2004 (2004-05-13) * paragraphs [0003] – [0005], [0018] – [0051]; figures 8a-8c * | 1-15 | INV. G05B23/02 G06Q10/00 G06N20/00 G16H40/40 |
| A | WO 2022/018104 A1 (KONINKLIJKE PHILIPS NV [NL]) 27 January 2022 (2022-01-27) * paragraphs [1to5], [0027], [0038], [0050]; claims 1-20 * | 1-15 | |
| A | US 2003/097054 A1 (SASAKI TAKUYA [JP] ET AL) 22 May 2003 (2003-05-22) * claims 1-8 * | 1-15 | |
| A | DE 10 2013 212972 A1 (SIEMENS AG [DE]) 28 May 2014 (2014-05-28) * paragraphs [0001] – [0014] * | 1-15 | |
| A | US 2019/019166 A1 (VAHID JAVID [US]) 17 January 2019 (2019-01-17) * paragraphs [0033] – [0035] * | 1-15 | TECHNICAL FIELDS SEARCHED (IPC) |
| A | EP 1 168 130 A1 (HITACHI LTD [JP]; HITACHI ENG SERVICE [JP]) 2 January 2002 (2002-01-02) * claims 1-10; figure 5b * | 1-15 | G06Q G05B G06N G06F G16H |

The present search report has been drawn up for all claims

| Place of search | Date of completion of the search | Examiner |
|---|---|---|
| Munich | 12 July 2022 | Juillot, Olivier J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 15 4269

This annex lists the patent family members relating to the patent documents cited in the above-mentioned European search report.
The members are as contained in the European Patent Office EDP file on
The European Patent Office is in no way liable for these particulars which are merely given for the purpose of information.

12-07-2022

| Patent document cited in search report | | Publication date | Patent family member(s) | | | Publication date |
|---|---|---|---|---|---|---|
| US 2004093184 | A1 | 13-05-2004 | AU | 2003267795 | A1 | 03-06-2004 |
| | | | CN | 1711546 | A | 21-12-2005 |
| | | | EP | 1563440 | A2 | 17-08-2005 |
| | | | JP | 2006505335 | A | 16-02-2006 |
| | | | US | 2004093184 | A1 | 13-05-2004 |
| | | | WO | 2004044814 | A2 | 27-05-2004 |
| WO 2022018104 | A1 | 27-01-2022 | NONE | | | |
| US 2003097054 | A1 | 22-05-2003 | NONE | | | |
| DE 102013212972 | A1 | 28-05-2014 | NONE | | | |
| US 2019019166 | A1 | 17-01-2019 | US | 2013275313 | A1 | 17-10-2013 |
| | | | US | 2019019166 | A1 | 17-01-2019 |
| EP 1168130 | A1 | 02-01-2002 | CA | 2338006 | A1 | 22-12-2001 |
| | | | CN | 1332422 | A | 23-01-2002 |
| | | | EP | 1168130 | A1 | 02-01-2002 |
| | | | JP | 3612472 | B2 | 19-01-2005 |
| | | | JP | 2002006942 | A | 11-01-2002 |
| | | | SG | 100630 | A1 | 26-12-2003 |
| | | | US | 2001056335 | A1 | 27-12-2001 |
| | | | US | 2005131656 | A1 | 16-06-2005 |

EPO FORM P0459

For more details about this annex : see Official Journal of the European Patent Office, No. 12/82